## Europäisches Patentamt

## European Patent Office

## Office européen des brevets

(19)

(11) Veröffentlichungsnummer: **0 043 494**
**B1**

(12)

# EUROPÄISCHE PATENTSCHRIFT

(45) Veröffentlichungstag der Patentschrift:
**16.05.84**

(21) Anmeldenummer: **81104850.3**

(22) Anmeldetag: **23.06.81**

(51) Int. Cl.³: **B 01 J 29/06,** B 01 J 21/06,
C 07 C 11/02, C 07 C 1/20

(54) **Verfahren zur Herstellung von niederen Olefinen aus Methanol und/oder Dimethylether.**

(30) Priorität: **28.06.80 DE 3024536**

(43) Veröffentlichungstag der Anmeldung:
**13.01.82 Patentblatt 82/2**

(45) Bekanntmachung des Hinweises auf die Patenterteilung:
**16.05.84 Patentblatt 84/20**

(84) Benannte Vertragsstaaten:
**BE DE FR GB IT NL**

(56) Entgegenhaltungen:
**EP - A - 0 002 492**
**DE - A - 2 015 505**
**DE - B - 1 145 152**
**US - A - 3 682 996**
**US - A - 4 062 905**
**US - A - 4 072 732**

(73) Patentinhaber: **HOECHST AKTIENGESELLSCHAFT,
Postfach 80 03 20, D-6230 Frankfurt am Main 80 (DE)**

(72) Erfinder: **Wunder, Friedrich, Dr., Jahnstrasse 46,
D-6093 Flörsheim am Main (DE)**
Erfinder: **Leupold, Ernst-Ingo, Dr., Am Zäunefeld 15,
D-6392 Neu-Anspach (DE)**
Erfinder: **Litterer, Heinz, Dr., Albert-Schweitzer-Allee 39,
D-6200 Wiesbaden (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Verfahren zur Herstellung von niederen Olefinen aus Methanol und/oder Dimethylether

In der DE-A-2 755 299, US-A-4 062 905, US-A-3 911 041, US-A-4 079 096, US-A-3 979 472 sowie DE-A-2 615 150 werden Verfahren zur Herstellung von niederen Olefinen aus Methanol und/oder Dimethylether an Aluminiumsilikat-Katalysatoren beschrieben. Allen diesen Katalysatoren gemeinsam ist, daß sie periodisch regeneriert, d. h. von abgelagerten Nebenprodukten befreit werden müssen, was schon bei relativ niedrigen Temperaturen von 300° C bis 500° C — am vorteilhaftesten bei der Reaktionstemperatur selbst — mit Luft erfolgen kann. Diese Regenerierung wird je nach Katalysator nach unterschiedlichen Zeiträumen notwendig, wobei die für niedere Olefine hoch spezifisch arbeitenden Katalysatoren verhältnismäßig kurze Laufzeiten zwischen den Regenerierungen haben, wogegen die ziemlich unspezifischen, einen breiten Bereich von Benzinkohlenwasserstoffen, Aromaten und Olefinen liefernden Katalysatoren oft längere Laufzeiten zwischen den Regenerierungen aufweisen. Somit stellte sich die Aufgabe, die hochselektiven Katalysatoren — die fast nur Ethylen und Propen liefern — so zu modifizieren, daß sie eine längere Laufzeit zwischen den Regenerierungen erreichen, wodurch ihr wirtschaftlicher Nutzen wesentlich gesteigert würde. Überraschenderweise kann dies durch einen geringen Zusatz von Hafnium- und/oder Zirkonionen erreicht werden. Aluminiumsilikatkatalysatoren, die Hafnium und/oder Zirkon (Zr) enthalten, sind um ein Vielfaches länger aktiv und bilden somit wesentlich mehr Ethylen und Propen aus Methanol und/oder Dimethylether als die gleichen Katalysatoren ohne den Zusatz von Hafnium und/oder Zirkon. Da Ethylen und Propen wichtige Chemierohstoffe und deshalb wesentlich wertvoller sind als Benzin, ist die Wertschöpfung bei diesen hochselektiven Katalysatoren besonders groß, wenn es gelingt, diesen Katalysatoren eine nahezu gleichbleibende Dauerleistung zu geben.

Die Erfindung betrifft ein Verfahren zur Herstellung von niederen Olefinen aus Methanol und/oder Dimethylether, das dadurch gekennzeichnet ist, daß man als Katalysator Aluminiumsilikat verwendet, auf das 0,1 bis 10 Gew.-% Hafnium oder Zirkon (Zr) oder beides aufgebracht wurden. Vorzugsweise enthält der Katalysator 0,5 bis 5 Gew.-% Hafnium oder Zirkon oder beides. Die Prozentangaben beziehen sich auf das Gesamtgewicht des fertigen Katalysators. Während die bisher bekannten Aluminiumsilikat-Katalysatoren für die Überführung von Methanol in niedere Olefine, besonders wenn sie hochaktiv und hochselektiv sind, nur eine Laufzeit von einigen Stunden haben, bis sie regeneriert werden müssen, verlängert sich die Laufzeit bei der Zugabe von Hafnium und/oder Zirkon auf ein Vielfaches.

Aus DE-B-1 145 152 und DE-A-2 015 505 sind Aluminiumsilikat-Katalysatoren bekannt, die Hafnium oder Zirkon (Zr) enthalten. Jedoch wird weder angegeben noch nahegelegt, daß derartige Katalysatoren bei der Synthese von niederen Olefinen aus Methanol und/oder Dimethylether eine besonders hohe Selektivität und eine besonders lange Laufzeit zwischen Regenerierungen zeigen. In der US-A-4 062 905 wird zwar die Verwendung eines Aluminiumsilikat-Katalysators für die genannte Synthese angegeben, unter Hinweis darauf, daß der Katalysator Metalle aus der Gruppe IV des Periodensystems enthalten kann, jedoch werden die Elemente von weiteren fünf Gruppen als gleichwertig angesehen. Aus derart allgemeinen Hinweisen kann der Gegenstand der vorliegenden Erfindung nicht hergeleitet werden.

Als Ausgangsmaterial für den Katalysator kommen beispielsweise die natürlichen oder synthetischen kristallinen Aluminiumsilikate in Frage, wie die unter den Bezeichnungen Faujasite, Zeolithe, Chabasite, Analcim, Gismondit, Gmelinit, Natrolith, Mordenite und Erionite bekannten Molekularsiebe. Vorteilhaft verwendet man das in großen Mengen natürlich vorkommende Chabasit-Erionitgemisch. Zum weiteren sind auch geeignet die üblichen, amorphen, sauren Crackkatalysatoren, die im allgemeinen zwischen 13 und 25 Gew.-% Aluminiumoxid und 75 bis 87 Gew.-% Kieselsäure enthalten.

Zur Herstellung der Katalysatoren werden die Aluminiumsilikate, ggf. vor oder nach — vorzugsweise nach — einer Stabilisierung, z. B. durch Auswaschen mit Ethylendiamintetraessigsäure oder durch Behandlung mit einer Silanverbindung, mit Hafnium- oder Zirkonionen beladen, so daß der fertige Katalysator 0,1 — 10 Gew.-%, vorzugsweise 0,5 — 5 Gew.-% Hafnium und/oder Zirkon enthält.

Die Hafnium- oder Zirkonionen können durch Imprägnieren des Aluminiumsilikats mit der entsprechenden Menge eines Hafnium- oder Zirkonsalzes oder durch partiellen Ionenaustausch aufgebracht werden. Bei einer Imprägnierung mit Hafnium- oder Zirkonsalzen ist vor dem Trocknen eine Alterungszeit des nassen Katalysators von einigen Tagen vorteilhaft. Als Hafnium- oder Zirkonsalze kommen alle löslichen Hafnium- oder Zirkonsalze in Betracht, beispielsweise Hafnium- oder Zirkonoxidchlorid, -bromid, sulfat, Hafnium- oder Zirkonoxalat, Hafnium- oder Zirkon-Alkali-Carbonate. Als Lösemittel wird i. allg. Wasser verwendet. Nach der Zirkon- oder Hafnium-Dotierung wird das Aluminiumsilikat vorzugsweise zusätzlich mit Mangan beladen, insbesondere durch einen Ionenaustausch, aber auch gegebenenfalls durch eine einfache Imprägnierung mit einem Mangansalz. Im Falle einer solchen Mangandotierung beträgt der Mangangehalt i. allg. 0,1 bis 10 Gew.-%. Er bewirkt eine Steigerung der Selektivität zu niederen Olefinen.

Als Lösemittel für die Mangansalze sind Wasser, Methanol, Formamid, Dimethylformamid oder auch deren Gemische bevorzugt, insbesondere Wasser. Als Mangansalze kommen alle leicht löslichen und gut zugänglichen Salze in Frage, z. B. das Formiat, Acetat, Propionat, Butyrat, Lactat, Citrat, Tartrat, Salz der Äpfelsäure, Chlorid, Bromid, Nitrat, Sulfat.

Es hat sich weiterhin oft als vorteilhaft für eine hohe Selektivität erwiesen, noch weitere Elemente als Co-Katalysatoren zu verwenden. Als solche sind Elemente geeignet, die in ihren Verbindungen ein-, zwei- oder dreiwertig vorkommen, wie beispielsweise die Alkalimetalle (insbesondere Lithium, Natrium und Kalium), die Erdalkalimetalle (insbesondere Magnesium, Calcium und Barium), Zink, Cadmium, Lanthan, Seltene Erden (wie Praseodym, Neodym, Samarium, Gadolinium oder auch ihre Mischungen wie Didymium) und Beryllium.

Diese co-katalytisch wirksamen Metallionen können gemeinsam oder nacheinander aufgetragen werden; bei gemeinsamer Lösung muß jedoch die gegenseitige Löslichkeitsbeeinflussung berücksichtigt werden, d. h. z. B. bei Einsatz von Calcium oder Barium sind Sulfate ungeeignet.

Die Aluminiumsilikate werden vorzugsweise zur Stabilisierung vor oder direkt nach dem Aufbringen von Hafnium und/oder Zirkon mit einer Lösung von Ethylendiamintetraessigsäure oder Weinsäure, die mit einer Base auf pH 3—7, vorzugsweise auf pH 4—5 eingestellt wurde, gewaschen. Als Basen eignen sich z. B. Lithiumhydroxid, Natriumhydroxid, Kaliumhydroxid, Rubidiumhydroxid und Caesiumhydroxid, besonders das Natriumhydroxid und das Kaliumhydroxid. Auch Alkali-Salze schwacher Säuren, wie die Carbonate eignen sich.

Die Konzentration der Lösungen von Ethylendiamintetraessigsäure oder Weinsäure können in weiten Grenzen variieren, von ca. 1%iger Lösung bis zu gesättigter Lösung; bevorzugt werden Lösungen, die bei Zimmertemperatur in etwa gesättigt sind. Die Temperatur dieser Lösungen liegt vorzugsweise zwischen 0°C und 50°C. Als Lösungsmittel sind bevorzugt Wasser, Methanol, Formamid, Dimethylformamid oder deren Gemische, insbesondere Wasser. Nach dem Auswaschen mit Ethylendiamintetraessigsäure- oder Weinsäure-Lösung wird der Katalysator zur Entfernung der Ethylendiamintetraessigsäure bzw. Weinsäure mit reinem Lösungsmittel gewaschen.

Bei der Herstellung von niederen Olefinen aus Methanol unter Einsatz des erfindungsgemäßen Katalysators kann man entweder Methanol direkt über den Katalysator leiten (wobei das Methanol einen hohen Wasseranteil enthalten kann) oder aber es zunächst an einem üblichen Dehydratisierungs-Katalysator, wie z. B. Aluminiumoxid oder Aluminiumsilikat, ganz oder teilweise in Dimethylether und Wasser überführen und den Ether dann alleine oder zusammen mit dem vorhandenen bzw. gebildeten Wasser und evtl. nicht umgesetztem Methanol über den Katalysator leiten. Man kann aber auch Gemische von Methanol und Dimethylether oder Dimethylether allein als Ausgangssubstanz verwenden.

Die Einsatzkomponenten Methanol und/oder Dimethylether können auch mit Inertgasen verdünnt in die Reaktion eingesetzt werden. Zur Erniedrigung des Partialdruckes sind beispielsweise Wasserstoff, Stickstoff und Kohlendioxid geeignet. Die Reaktion kann aber zu diesem Zweck auch bei vermindertem Druck bis herab zu 0,1 bar durchgeführt werden.

Der Wassergehalt der Einsatzprodukte kann in weiten Grenzen schwanken, von wasserfrei bis zu etwa 80% Wasser, wobei jedoch höhere Wassermengen höhere Verdampfungs- und Destillationskosten hervorrufen, so daß die Obergrenze des Wassergehaltes von wirtschaftlichen Überlegungen bestimmt wird.

Die Reaktionstemperatur liegt im allgemeinen zwischen 300°C und 500°C, bevorzugt zwischen 380°C und 420°C. Wählt man die Reaktionsbedingungen so, daß nur ein unvollständiger Umsatz an Methanol und/oder Dimethylether erreicht wird, so können die nicht umgesetzten Anteile abgetrennt und zurückgeführt werden. Die an den Katalysatoren hergestellten Alkene können nach den üblichen Methoden, z. B. durch Destillation, von den als Nebenprodukten entstehenden Alkanen und voneinander getrennt werden.

Damit steht ein Katalysator zur Verfügung, der in besonders selektiver und damit wirtschaftlicher Weise die Herstellung von industriell bedeutenden niederen Alkenen aus Methanol und/oder Dimethylether in Gegenwart von großen Mengen Wasser gestattet, wobei die Laufzeiten des Katalysators zwischen den Regenerierungen ein Vielfaches der üblichen Katalysatoren betragen. Hierdurch erhöht sich die durchschnittliche Raum-Zeitleistung dieser hochspezifisch Ethylen und Propen erzeugenden Katalysatoren wesentlich.

Die folgenden Beispiele zeigen die Verwendung des Katalysators.

Vergleichsbeispiel 1

Es werden 300 ml eines handelsüblichen Chabasit-Erionit-Gemisches in Form von Strangpreßlingen mit 300 ml gesättigter wäßriger Manganacetat-Lösung überschichtet, nach 48 Stunden mit Wasser ausgewaschen und getrocknet. Man erhält 202 g Katalysator mit 3,6 Gew.-% Mangan. Über diesen Katalysator werden stündlich 89,1 g Methanol bei 1 bar und 400°C geleitet. Man erhält 25 l Gas je Stunde, bestehend aus

26,6 Gew.-% Ethylen
27,9 Gew.-% Propylen
4,6 Gew.-% Butene
6,7 Gew.-% Methan
1,3 Gew.-% Ethan
17,4 Gew.-% Propan
3,0 Gew.-% Butan

0,5 Gew.-% Sonstiges
12,0 Gew.-% Dimethylether

sowie 9,2 g Methanol und 43,3 g Wasser. Dies entspricht einem 89,6%igen Umsatz von Methanol, einer Selektivität zu $C_2-C_4$-Olefinen von 68,9%, und einer Selektivität zu $C_2-C_4$-Kohlenwasserstoffen von 93%, wenn der gebildete Dimethylether und das nicht umgesetzte Methanol zurückgeführt werden. Nach 6 Stunden ist bei gleicher Selektivität die Leistung abgesunken, wobei der Anteil an Dimethylether entsprechend ansteigt.

Vergleichsbeispiel 2

Man wiederholt Vergleichsbeispiel 1 mit dem einzigen Unterschied, daß dem Einsatz-Methanol Wasser zugegeben wird. Man erhält bei einem Einsatz von stündlich 87,4 g Methanol und 43,1 g Wasser 26 l/h eines Gases mit

30,0 Gew.-% Ethylen
29,4 Gew.-% Propen
5,4 Gew.-% Butene
7,3 Gew.-% Methan
1,2 Gew.-% Ethan
13,2 Gew.-% Propan
2,2 Gew.-% Butan
0,3 Gew.-% Sonstiges
11,3 Gew.-% Dimethylether

sowie 7,8 g Methanol und 86,0 g Wasser. Dies entspricht einem Methanol-Umsatz von 91,1%, einer Selektivität zu $C_2-C_4$-Olefinen von 74,2%, und einer Selektivität zu $C_2-C_4$-Kohlenwasserstoffen von 92,5%, wenn Dimethylether und nicht umgesetztes Methanol zurückgeführt werden. Nach 5 Stunden muß der Katalysator wegen nachlassender Leistung regeneriert werden und hat dann wieder dieselbe Leistung wie zu Beginn.

Beispiel 1 (mit Hafnium)

Es werden 200 g eines mit 5% Tonerde gebundenen Chabasit-Erionit-Gemisches mit einer gesättigten Lösung des Di-Natriumsalzes der Ethylendiamintetraessigsäure überschichtet, 48 Stunden stehen gelassen, mit dest. Wasser ausgewaschen und getrocknet. Dann werden 4 g Hafniumoxidchloridhydrat ($HfOCl_2 \cdot 8 H_2O$) in 70 ml Wasser gelöst, der Katalysator wird mit dieser Lösung getränkt, 48 Stunden bei Zimmertemperatur stehen gelassen und dann getrocknet. Anschließend wird er mit einer Lösung von 9 g Manganacetathydrat in 75 ml Wasser getränkt und getrocknet. Der fertige Katalysator enthält 1 Gew.-% Mangan und 1 Gew.-% Hafnium.

Über diesen Katalysator werden bei 400° C und 1 bar stündlich 320 ml Methanol/Wasser (50 : 50) geleitet. Man erhält 37 l/h Gas, das folgende Zusammensetzung hat:

34,8 Gew.-% Ethylen
33,2 Gew.-% Propen
7,4 Gew.-% Butene
3,1 Gew.-% Methan
0,9 Gew.-% Ethan
4,6 Gew.-% Propan
2,6 Gew.-% Butan
1,9 Gew.% Sonstiges
11,5 Gew.-% Dimethylether

sowie 222 g Wasser und 5,7 g Methanol. Dies entspricht einer Selektivität von

40,2% zu Ethylen
38,4% zu Propen
8,6% zu Butenen

$C_2-C_4$-Olefine entstehen mit insgesamt 87,2% Selektivität, $C_2-C_4$-Kohlenwasserstoffe mit 96,5% Selektivität, wenn der Dimethylether zurückgeführt wird.

Nach 8 Stunden Laufzeit haben sich die Leistungen praktisch nicht geändert.

Beispiel 2 (mit Zirkon)

Es werden 210 g eines handelsüblichen Chabasit-Erionit-Gemisches in Form von 1,5 mm Strangpreßlingen mit einer 10%igen Lösung von Ethylendiamintetraessigsäure-Di-Natriumsalz behandelt, mit Wasser ausgewaschen und getrocknet. Dann werden 0,8 g Zirkonylchlorid in 70 ml Wasser gelöst, der Katalysator wird mit dieser Lösung imprägniert und nach einer Ruhezeit von 62 Stunden getrocknet. Anschließend wird er mit einer Lösung von 9, 5 g Manganacetat in 70 ml Wasser imprägniert und getrocknet.

Der fertige Katalysator enthält 0,1 Gew.-% Zirkon und 1 Gew.-% Mangan.

Über diesen Katalysator werden bei 400° C und 1 bar stündlich 300 ml eines Gemisches aus zwei Gewichtsteilen Methanol und einem Gewichtsteil Wasser geleitet.

Innerhalb einer Stunde erhält man 49 l eines Gases, bestehend aus

36,1 Gew.-% Ethylen
37,3 Gew.-% Propen
5,8 Gew.-% Butene
2,5 Gew.-% Methan
0,9 Gew.-% Ethan
3,7 Gew.-% Propan
2,8 Gew.-% Butan
1,4 Gew.-% Sonstiges
9,5 Gew.-% Dimethylether

sowie 181 g Wasser und 6,9 g Methanol, entsprechend einer Selektivität von

40% zu Ethylen
41,3% zu Propen
6,4% zu Butenen

einer Selektivität zu $C_2-C_4$-Olefinen von 87,7%

und zu $C_2-C_4$-Kohlenwasserstoffen von 97%.

Nach 8 Stunden Laufzeit hat die Ethylenleistung unwesentlich nachgelassen.

### Beispiel 3 (mit Hafnium)

Es werden 210 g eines handelsüblichen Chabasit-Erionit-Gemisches als 1 mm Strangpreßlinge mit einer 10%igen Lösung von Dinatriumethylendiamintetraessigsäure in Wasser gründlich gewaschen, mit Wasser nachgewaschen und getrocknet. Dieser Katalysator wird mit einer gesättigten Lösung von Hafniumoxidchlorid überschichtet, nach 48 Stunden mit Wasser ausgewaschen, getrocknet und mit einer Lösung von 9 g Manganacetat in 70 ml Wasser getränkt und anschließend getrocknet. Der fertige Katalysator enthält 8,3% Hafnium und 1% Mangan.

Über diesen Katalysator werden bei 400° C und 1 bar stündlich 300 ml eines Gemisches aus zwei Teilen Methanol und einem Teil Wasser geleitet.

Innerhalb einer Stunde erhält man 51 l eines Gases, bestehend aus

12,7 Gew.-% Ethylen
8,3 Gew.-% Propen
3,6 Gew.-% Butene
1,8 Gew.-% Methan
0,8 Gew.-% Ethan
4,8 Gew.-% Propan
2,4 Gew.-% Butan
64,4 Gew.-% Dimethylether
1,2 Gew.-% Sonstiges

sowie 147 g Wasser und 16,3 g Methanol.
Dies entspricht einer Selektivität von

36,0% zu Ethylen
23,5% zu Propen
10,2% zu Butenen

Die $C_2-C_4$-Olefine entstehen mit 69,7% Selektivität, die $C_2-C_4$-Kohlenwasserstoffe mit 92,6% Selektivität, wenn der Dimethylether zurückgeführt wird.

Nach 8 Stunden Laufzeit sind diese Werte praktisch unverändert.

### Patentansprüche

1. Verfahren zur Herstellung von niederen Olefinen aus Methanol und/oder Dimethylether, dadurch gekennzeichnet, daß man als Katalysator Aluminiumsilikat verwendet, auf das 0,1 bis 10 Gew.-% Hafnium oder Zirkon (Zr) oder beides aufgebracht wurden.

2. Verfahren nach Anspruch 1, dadurch gekennzeichnet, daß auf das Aluminiumsilikat 0,5 bis 5 Gew.-% Hafnium oder Zirkon oder beides aufgebracht wurden.

3. Verfahren nach Anspruch 1 oder 2, dadurch gekennzeichnet, daß nach Hafnium und/oder Zirkon noch Mangan auf das Aluminiumsilikat aufgebracht wurde.

4. Verfahren nach einem der Ansprüche 1 bis 3, dadurch gekennzeichnet, daß das Aluminiumsilikat vor dem Aufbringen von Hafnium und/oder Zirkon durch eine Behandlung mit Ethylendiamintetraessigsäure oder einer Silanverbindung stabilisiert wurde.

### Claims

1. Process for the preparation of lower olefins from methanol and/or dimethyl ether, characterized by using as a catalyst aluminum silicate to which 0,1 to 10% by weight of hafnium or zirconium or both have been applied.

2. Process as claimed in claim 1, wherein 0,5 to 5% by weight of hafnium or zirconium or both have been applied to the aluminum silicate.

3. Process as claimed in either of claims 1 or 2, wherein, after hafnium and/or zirconium, manganese has also been applied to the aluminum silicate.

4. Process as claimed in any one of claims 1 to 3, wherein, before the hafnium and/or zirconium has been applied, the aluminum silicate has been stabilized by means of a treatment with ethylenediaminetetraacetic acid or a silane compound.

### Revendications

1. Procédé de préparation d'oléfines inférieures à partir de méthanol et/ou d'oxyde de diméthyle, procédé caractérisé en ce qu'on utilise, comme catalyseur, un silicate d'aluminium sur lequel on a déposé de 0,1 à 10% en poids d'hafnium ou de zirconium ou d'un ensemble des deux.

2. Procédé selon la revendication 1 caractérisé en ce qu'on applique, sur le silicate d'aluminium, de 0,5 à 5% en poids d'hafnium ou de zirconium ou d'un ensemble des deux.

3. Procédé selon l'une des revendications 1 et 2, caractérisé en ce qu'après l'hafnium et/ou le zirconium on applique du manganèse sur le silicate d'aluminium.

4. Procédé selon l'une quelconque des revendications 1 à 3, caractérisé en ce qu'avant d'appliquer l'hafnium et/ou le zirconium on stabilise le silicate d'aluminium en le traitant par l'acide éthylène-diamine-tétracétique ou par un dérivé du silane.